# FASCICULE DE BREVET EUROPEEN

(11) **EP 1 318 829 B1**
(45) Date de publication et mention de la délivrance du brevet: **20.04.2005**
(21) Numéro de dépôt: 01972173.7
(22) Date de dépôt: 20.09.2001
(51) Int. Cl.: A61K 38/07, C12N 5/00, A61P 9/10, A61P 17/02, A61P 41/00

(54) **Tri- ou tétrapeptides angiogéniques dérivés de AcSDKP**
Angiogenische Tri- oder Tetrapeptide abgeleitet von AcSDKP
Angiogenic tri- or tetrapeptides derived from AcSDKP

(30) Priorité: 21.09.2000 FR 0012028
(43) Date de publication de la demande: 18.06.2003
(73) Titulaire: CENTRE NATIONAL DE LA RECHERCHE SCIENTIFIQUE (CNRS), 75794 Paris Cedex 16 (FR)
(72) Inventeur: BAKALA, Joanna, F-75012 Paris (FR); POTIER, Pierre, Jean-Paul, F-75007 Paris (FR); LAWRENCE, Françoise, F-91470 Boullay-Les-Troux (FR); CHEVIRON, Nathalie, F-91470 Limours (FR); BIGNON, Jérôme, F-91530 Le Val St-Germain (FR); FROMES, Yves, F-75015 Paris (FR)
(74) Mandataire: Warcoin, Jacques
(86) Numéro de dépôt international: PCT/FR2001/002923
(87) Numéro de publication internationale: WO 2002/024218

(56) Documents cités:
- WO-A-00/06190
- WO-A-97/28183
- US-A- 5 470 831
- HUFF T ET AL.: ".beta.-Thymosins, small acidic peptides with multiple functions" THE INTERNATIONAL JOURNAL OF BIOCHEMISTRY & CELL BIOLOGY, vol. 33, no. 3, 2001, pages 205-220, XP001009478

## Description

La présente invention concerne des composés induisant l'angiogenèse et leurs applications, notamment dans le traitement de certaines pathologies vasculaires.

L'angiogenèse est un phénomène physiologique fondamental présent tout au long de la vie d'un individu et qui permet de maintenir l'intégrité structurale et fonctionnelle de l'organisme. L'angiogenèse apparaît en réponse à des stimuli locaux qui induisent une cascade d'événements conduisant à la formation de nouveaux vaisseaux sanguins à partir de vaisseaux préexistants.

Au cours d'une néovascularisation, les cellules endothéliales représentent l'élément central indispensable à la création de nouveaux vaisseaux. Leur développement et leur croissance sont régulés par des facteurs positifs (angiogéniques) et négatifs (angiostatiques). Au niveau des vaisseaux déjà formés, les cellules endothéliales sont à l'état quiescent. Cependant, en réponse à un stimulus angiogénique, ces cellules se mettent à proliférer et à migrer vers le lieu de création des nouveaux vaisseaux ce qui nécessite la création d'interactions avec les cellules environnantes et avec les éléments de la matrice extracellulaire.

Chez l'adulte, l'angiogenèse est un processus clé des fonctions reproductrices (formation du corps jaune, formation du placenta, développement de l'endomètre) et surtout de la réparation tissulaire lors de traumatismes (cicatrisation) et d'ischémies. Il est donc évident que la stimulation de l'angiogenèse par l'administration de molécules exogènes représenterait un progrès important dans la thérapeutique de certaines affections telles que la réparation de plaies cutanées, osseuses, gastriques ou ophtalmiques, par exemple. Les perspectives d'utilisation clinique de tels médiateurs angiogéniques sont également ouvertes pour favoriser la régénération tissulaire associée aux pathologies ischémiques ou pour l'endothélialisation de prothèses.

Plusieurs facteurs angiogéniques, capables d'induire *in vitro* comme *in vivo* les étapes de l'angiogenèse ont été identifiés (angiogénine, angiopoïétine, interleukine 8 (IL-8), « epidermal growth factor » (EGF), « fibroblast growth factors » (FGFs), « transforming growth factor » (TGF) α et β, « hepatocyte growth factor » (HGF), « Plateled-derived endothelial growth factor » (PDGF), « tumor necrosis factor α » (TNFα), « vascular endothelial growth factors » (VEGFs), « placental growth factor » (PIGF). Tous ces facteurs, de nature protéique, font l'objet de recherches intenses sur leurs aptitudes à la réparation tissulaire chez l'homme. Les essais cliniques en phase I et II avec les VGEFs et les FGFs ont été débutés. En particulier, ces facteurs sont évalués pour leurs effets thérapeutiques sur les pathologies ischémiques aussi bien cardiaque que cérébrale. Les résultats actuels de ces essais montrent que quel que soit le mode d'administration de ces facteurs (perfusion des protéines recombinantes ou thérapie génique), ces derniers ne manifestent pas l'efficacité thérapeutique attendue (Ferrara N. Alitalo K., 1999 Clinical applications of angiogenic growth factors and their inhibitors. *Nature Med*., 5 : 1359-1364). De nouvelles voies visant à stimuler la néovascularisation tissulaire se dirigent actuellement vers des thérapies combinant plusieurs facteurs angiogéniques.

La disponibilité des facteurs angiogéniques sous forme de protéines recombinantes a permis de montrer que leur application locale pouvait accélérer la guérison des plaies. Le produit qui semble le plus intéressant est le TGFβ. Ses propriétés cicatrisantes sont solidement établies depuis plusieurs années et des essais cliniques sont en cours.

En tout état de cause, la plupart de ces facteurs angiogéniques sont des protéines de haut poids moléculaire qui ne peuvent être obtenues que par voie recombinante, ce qui par là même augmente le prix de revient du traitement.

Si certains polypeptides sont susceptibles de stimuler l'angiogenèse, les inventeurs ont réussi à démontrer une activité angiogénique propre des structures peptidiques de petite taille, et plus spécialement de quatre acides aminés.

On notera que les peptides de petite taille, incluant la formule I ou au moins les trois acides aminés (comprenant les radicaux correspondant à A₁, A₂, A₃), font également partie de l'invention.

C'est pourquoi la présente invention concerne, pour ce type d'application, l'utilisation de petits peptides dont la synthèse chimique ne pose pas de problème et dont le prix de revient est assez bas.

En outre, l'utilisation de ces peptides présente, sur le plan industriel, un certain nombre d'avantages parmi lesquels, notamment, le fait qu'ils sont de manipulation plus aisée que des protéines.

La présente invention concerne l'utilisation d'un composé de formule I : 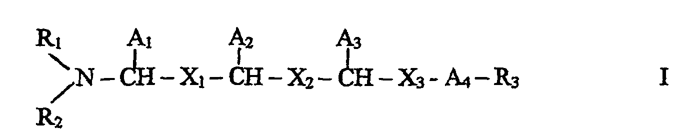 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi H, C₁-C₁₂-alkyle substitué ou non, C₇-C₂₀-arylalkyle substitué ou non, R₄CO ou R₄COO, R₄ étant C₁-C₁₂-alkyle substitué ou non, C₇-C₂₀-arylalkyle substitué ou non, parmi les substitutions il faut citer OH, NH₂ ou COOH,
X₁ et X₂ sont des liaisons peptidiques ou pseudopeptidiques,
X₃ est CO ou CH₂ et
R₃ est OH, NH₂, C₁-C₁₂-alcoxy ou NH-X₄-CH₂-Z, X₄ est un C₁-C₁₂-hydrocarbone normal ou ramifié, Z est H, OH, CO₂H ou CONH₂,
ou bien les tripeptides correspondants comprenant les radicaux A₁, A₂, A₃,
ainsi que les sels pharmaceutiquement acceptables, pour la réalisation d'un médicament destiné au traitement des pathologies pouvant bénéficier d'une angiogenèse.

Par l'expression « bénéficier d'une angiogenèse » on entend bénéficier d'une induction et/ou d'une stimulation de l'angiogenèse.

Les peptides ou pseudopeptides correspondant à ces formules sont dérivés de la structure de base du tétrapeptide Acétyl-Ser-Asp-Lys-Pro (AcSDKP), ces dérivés étant destinés notamment à augmenter l'activité angiogénique, diminuer les effets secondaires et/ou augmenter la durée de vie en milieu physiologique.

Parmi les composés de l'invention il faut mentionner le tripeptide dérivé d'un peptide AcSDKP et comprenant les radicaux A₁, A₂, A₃.

La structure de base est une molécule qui a été isolée de la moelle osseuse de veau foetal et dont les applications ont, jusqu'à présent, été liées à une fonction de l'inhibition de la prolifération des cellules souches hématopoïétiques, décrite notamment dans WO 88/00594.

Par « radical correspondant à » il faut entendre le radical A de la formule : NH₂-CH(A)-COOH correspondant à l'acide aminé.

Ainsi, A est
-CH₂OH pour Ser,
-CH₂COOH pour Asp,
-CH₂-CH₂-COOH pour Glu,
-(CH₂)₃-NH-C(NH)NH₂ pour Arg,
-(CH₂)₃-NH₂ pour Orn et
-(CH₂)₄-NH₂ pour Lys,
pour l'acide aminé terminal A₄, il s'agit soit de la structure :
=N-CH(A)-CO- ou NH-(CH)A-CO-.

Par « pseudopeptide » on entend désigner des composés similaires aux peptides de référence mais dans lesquels une ou plusieurs liaisons peptidiques -CO-NH- ont été remplacées par une liaison équivalente à la liaison peptidique qui est appelée pseudopeptidique, soit -CH₂-NH-, -CH₂-S-, -CH₂-O-, -CO-CH₂-, -CH₂-CO-, -CH₂-CH₂- représenté par Ψ (CH₂NH) par exemple.

Parmi les radicaux R₁ et R₂, on préférera tout particulièrement les radicaux: H et (C₁-C₃)-alkyl-CO- notamment CH₃CO ainsi que HOOC-CH₂-CH₂-CO-O.

De même, R₃ est de préférence NH₂, OH ou NHCH₃.

Parmi les composés, il faut citer :
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

Ainsi que les composés suivants :
CH₃CO-Ser-Asp-Lys-OH
CH₃CO-Ser-Ψ-(CH₂NH)-Asp-Lys-OH
CH₃CO-Ser-Asp-Ψ-(CH₂NH)-Lys-OH
CH₃CO-Ser-Ψ-(CH₂NH)-Asp-Lys-NH₂
CH₃CO-Ser-Asp-Ψ-(CH₂NH)-Lys-NH₂
H-Ser-Ψ-(CH₂NH)-Asp-Lys-OH
H-Ser-Asp-Ψ-(CH₂NH)-Lys-OH
HOOCCH₂CH₂CO-Ser-Ψ-(CH₂NH)-Asp-Lys-OH
HOOCCH₂CH₂CO-Ser-Asp-Ψ-(CH₂NH)-Lys-OH
H-Ser-Ψ-(CH₂NH)-Asp-Lys-NH₂
H-Ser-Asp-Ψ-(CH₂NH)-Lys-NH₂
HOOCCH₂CH₂CO-Ser-Ψ-(CH₂NH)-Asp-Lys-NH₂
HOOCCH₂CH₂CO-Ser-Asp-Ψ-(CH₂NH)-Lys-NH₂
CH₃CO-Ser-Asp-Lys-NH₂
H-Ser-Asp-Lys-NH₂
CH₃CO-Ser-Asp-Lys-NHCH₃
H-Ser-Asp-Lys-NHCH₃
HOOCCH₂CH₂CO-Ser-Asp-Lys-NHCH₃
HOOCCH₂CH₂CO-Ser-Asp-Lys-NH₂

Parmi les sels pharmaceutiquement acceptables, il faut citer notamment les sels avec des acides non organiques, chlorure, sulfate, phosphate, nitrate, chlorhydrate, mais également avec les acides organiques, notamment lactate, citrate par exemple, ainsi que les sels avec les bases.

Les inventeurs ont maintenant mis en évidence que ce tétrapeptide ou des peptides apparentés de formule I étaient capables d'induire l'angiogenèse.

Parmi les traitements de pathologies pouvant bénéficier d'une stimulation de l'angiogenèse, il faut citer les pathologies vasculaires, notamment dans le traitement des ischémies. Ainsi, les composés selon la présente invention peuvent être utilisés notamment dans les cas suivants :
1. induction de la formation de vaisseaux collatéraux dans le cas des pathologies ischémiques :
   - ischémie myocardique (maladie des artères coronaires, infarctus du myocarde),
   - ischémie périphérique (occlusion des artères périphériques),
   - ischémies cérébrales (maladies vasculaires cérébrales),
2. cicatrisations et réparations de fractures dans le cas des lésions des tissus :
   - dermatologie : brûlures ou blessures, ulcères chroniques,
   - ophtalmologie: lésions cornéennes ou rétiniennes,
   - gastro-entérologie : ulcères gastroduodénaux,
   - chirurgie des os : réparations des tissus durs : os + cartilage,
3. régénération nerveuse,
4. chirurgie reconstructive, et chirurgie esthétique,
5. endothélialisation des biomatériaux et d'implants vasculaires,
6. transplantation d'organes (par exemple les îlots de Langherans).

La liste précédente ne constitue qu'une partie des applications possibles, notamment dans le cas de traitements combinés, une accélération de l'angiogenèse peut permettre d'accélérer la guérison.

Les composés selon la présente invention sont également utiles dans les cultures *ex vivo* ou *in vitro* de tissus nécessitant une néovascularisation comme inducteurs d'angiogenèse, par exemple, dans la culture de peau ou dans les enrobages de matériaux par des tissus.

*In vitro* on a pu montrer que AcSDKP est capable de stimuler significativement la croissance des cellules endothéliales humains (HUVEC et EA.hy926) et des cellules endothéliales provenant des capillaires de cerveau bovin (BBC).

*In vivo*, l'activité angiogénique de AcSDKP a été testée sur le modèle expérimental « de la membrane chorioallantoïdienne de l'embryon de poulet ». Sur ce modèle, AcSDKP induit de façon importante la néovascularisation.

D'autre part, une étude préliminaire réalisée sur des cellulaires médullaires a révélé que AcSDKP *in vitro* augmente l'adhérence de ces cellules sur différents composants de la matrice extracellulaire telles que la fibronectine, le collagène IV et la laminine. Des résultats comparables ont été obtenus dans une expérience préliminaire réalisée avec des cellules endothéliales humaines HUVEC. Il est bien établi que ces interactions entre des cellules endothéliales et la matrice extracellulaire sont déterminantes pour la néovascularisation et interviennent à différentes étapes de l'angiogenèse. En effet, la matrice extracellulaire influence la prolifération et la migration des cellules endothéliales vasculaires, ainsi que leur capacité à se différencier et à s'organiser en capillaires pour former de nouveaux vaisseaux fonctionnels adaptés à leur micro-environnement tissulaire.

Les composés selon la présente invention peuvent être administrés sous une forme pharmaceutique appropriée, par exemple par voie orale, intraveineuse, transdermique, pulmonaire, sous-cutanée, nasale ou autres, avec les formes correspondantes, qu'il s'agisse de comprimés, de solutions injectables ou de pommades, de gels, notamment lorsque l'on souhaitera réaliser des compositions destinées à améliorer la cicatrisation.

Dans les revascularisations, notamment dans le traitement des ischémies, on aura de préférence recours à des voies injectables et en particulier à des perfusions.

Bien entendu, les composés selon la présente invention pourront être utilisés en combinaison avec d'autres principes actifs destinés éventuellement à traiter directement la pathologie lorsque l'angiogenèse ne constituera qu'une thérapie d'appoint à une thérapie principale, par exemple dans le traitement des ulcères gastroduodénaux.

Pour ce qui concerne les doses d'administration, dans les modèles utilisés on a constaté que l'effet angiogénique passait par un maximum pour ensuite redécroître si on augmentait les doses administrées. Il faudra donc éventuellement adapter la dose d'administration au type de pathologie et au patient si cela est nécessaire. Les doses optimales de AcSDKP se situent entre 10⁻⁵ et 10⁻¹¹ M et seront de préférence situées entre 10⁻⁶ et 10⁻⁹.

Les composés selon la présente invention peuvent être synthétisés par des procédés de synthèse de type peptidique ou pseudopeptidique, notamment les procédés décrits dans WO 88/00594 qui décrit la synthèse du dérivé AcSDKP et dans le brevet WO 97/28183 qui décrit la synthèse de pseudopeptides apparentés à AcSDKP.

Légende des figures :
- Figures 1a, 1b et 1c : AcSDKP stimule *in vitro* la pousse des cellules endothéliales bovines (BBC) et humaines (HUVEC et EA.hy926).
- Figures 2a, 2b et 2c : effet de AcSDKP et de ses analogues sur la vascularisation de la membrane chorioallantoïdienne d'embryon de poulet (CAM).
- Figure 3 : effet de AcSDKP sur la formation des tubes vasculaires *in vitro* par les cellules endothéliales (EA.hy 926) dans le Matrigel.

### EXEMPLE 1

Les essais suivants ont été réalisés avec le tétrapeptide AcSDKP, on a observé les résultats suivants :

AcSDKP stimule significativement la croissance des cellules endothéliales provenant de capillaires de cerveau bovin (BBC) et des cellules endothéliales veineuses de cordons ombilicaux humains (HUVEC) ainsi que la croissance d'une lignée de cellules HUVEC immortalisées (EA.hy926). L'effet mitogène de AcSDKP se manifeste à des concentrations variant entre 10⁻⁶ et 10⁻¹¹ M avec un maximum pour 10⁻⁹ M.

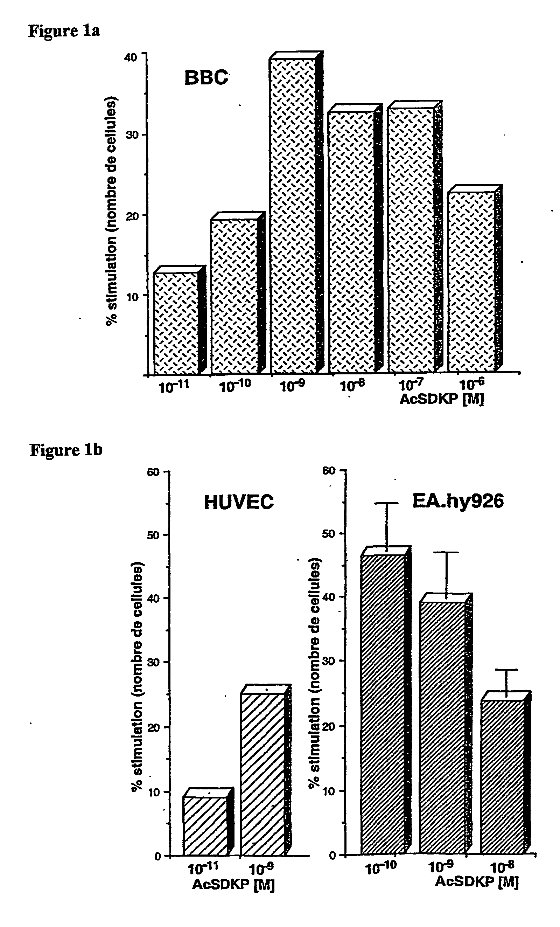

Les résultats de ces études sont rassemblés sur les figures 1a, 1b et 1c.

Une étude complémentaire réalisée sur de la moelle totale (contenant entre autre des cellules endothéliales) a montré que AcSDKP *in vitro* augmente l'adhérence des cellules de moelle sur divers composants de la matrice extracellulaire telles que la fibronectine, le collagène IV et la laminine.

La concentration optimum de AcSDKP dans ce type de modèle se situe entre 10⁻⁶ et 10⁻⁸ M.

Des résultats comparables ont été obtenus dans des expériences préliminaires réalisées sur cellules endothéliales humaines HUVEC.

Il est bien établi que ces interactions entre les cellules endothéliales et la matrice extracellulaire sont déterminantes pour la néovascularisation et interviennent à différentes étapes de l'angiogenèse. En effet, la matrice extracellulaire influence la prolifération et la migration des cellules endothéliales vasculaires, ainsi que leur capacité à se différencier et à s'organiser en capillaires pour former de nouveaux vaisseaux fonctionnels adaptés à leur micro-environnement tissulaire.

### EXEMPLE 2

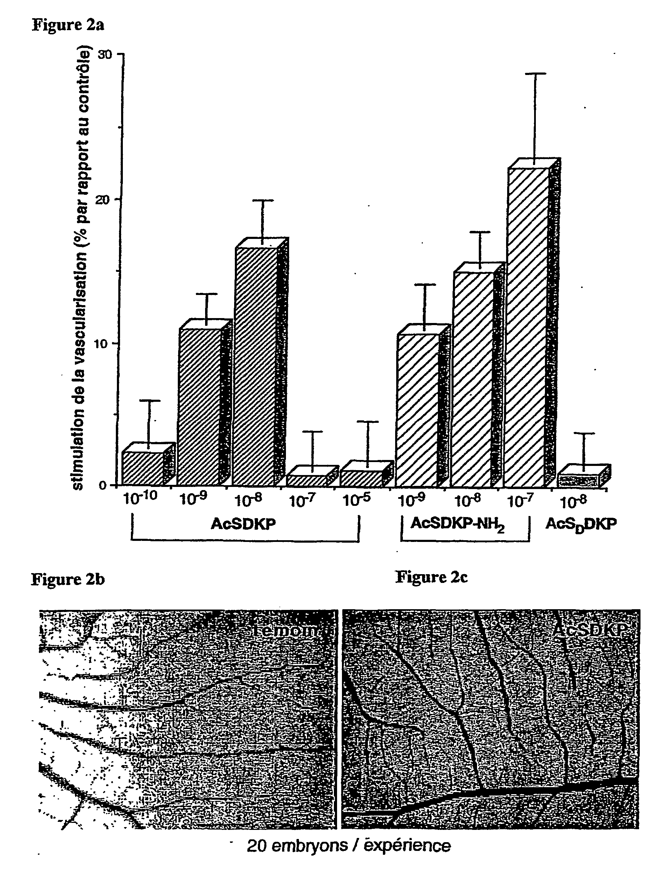

Les expériences suivantes ont été réalisées sur le modèle expérimental de la membrane chorioallantoïdienne de l'embryon de poulet et sur ce modèle on a testé l'activité de AcSDKP et de ses analogues. On a pu démontrer que AcSDKP et son analogue résistant à la protéolyse, AcSDKP-NH₂, induisaient de façon significative la néovascularisation, alors que son isomère optique, AcS_{D}DKP, n'avait aucun effet angiogénique. L'augmentation de la densité de la vascularisation varie en fonction de la dose étudiée avec un maximum d'effet pour AcSDKP et AcSDKP-NH₂ aux concentrations comprises entre 10⁻⁹M et 10⁻⁷ (figures 2a, 2b et 2c).

### EXEMPLE 3

Les résultats d'une étude *in vivo* réalisée chez le rat montrent que AcSDKP induit également la néovascularisation chez les mammifères.

Dans ces expériences, AcSDKP a été injecté dans le muscle abdominal chez le rat normal (traitement deux fois par jour pendant 5 jours). Une angiographie des parois abdominales réalisée chez l'animal sacrifié le jour 8 a révélé une vascularisation plus développée (augmentation significative du nombre des petits vaisseaux) chez des animaux traités avec AcSDKP à la dose 5 µg/kg/injection. Il faut souligner que cet effet persiste dans le temps (observations réalisées un mois après le début du traitement). Aucune modification significative de la vascularisation n'a été observée suite à l'administration de AcSDKP à la dose de 50 µg/kg/injection.

La perte de l'activité angiogénique de AcSDKP liée avec l'augmentation de la dose administrée que l'on a observée aussi bien *in vitro* (cellules en culture) qu'*in vivo* (embryon de poulet et rat) reste en accord avec l'existence pour AcSDKP d'une dose-réponse en cloche comme précédemment décrite [(Guignon M. Bonnet D, Lemoine F., Kobari L., Parmentier C., Mary JY, Najman A. (1990) Inhibition of human bone marrow progenitors by the synthetic tetrapeptide AcSDKP ; Exp. Hematol. 18, 1112 ; Jackson JD, Yan Y., Ewel C., Talmage JE. (1996) Activity of Acetyl-Ser-Asp-Lys-Pro (AcSDKP) on hematopoietic progenitors in short term and long-term murine bone marrow cultures. Exp. Hematol., 24, 475)].

### EXEMPLE 4

L'organisation des cellules endothéliales en tubes vasculaires constitue une étape importante de l'angiogenèse. On a montré que AcSDKP stimule, aussi bien *in vitro* qu'*in vivo*, la formation des tubes dans le Matrigel.

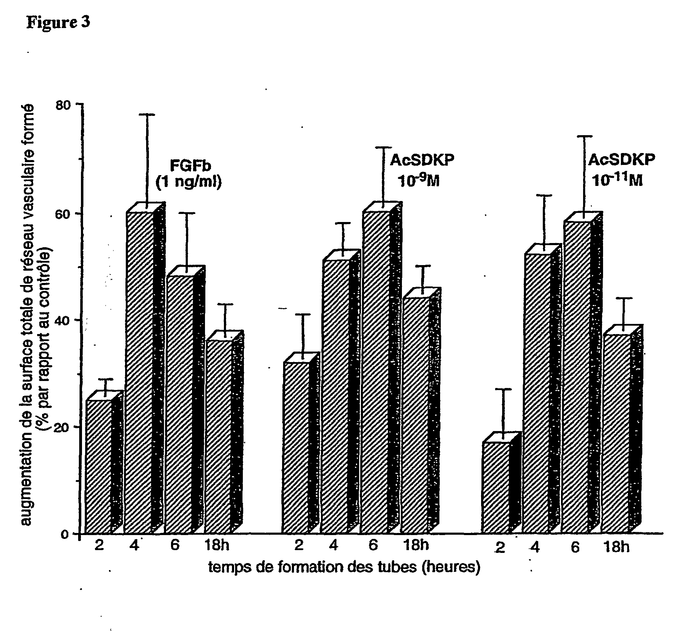

Les expériences réalisées *in vitro* avec deux types de cellules endothéliales montrent que AcSDKP aux concentrations de 10⁻⁹M et 10⁻¹¹M induit une augmentation de la surface totale du réseau vasculaire par rapport aux valeurs contrôles. Les stimulations maximales correspondant respectivement à 60% et 58% d'augmentation ont été observés après 6 heures d'exposition des cellules au tétrapeptide. Ces effets sont comparables à celui généré par le FGFb à 1 ng/ml (figure 3).

*In vivo*, AcSDKP induit de façon dose-dépendante l'invasion vasculaire du Matrigel placé sous la peau de l'animal (rat Sprague-Dawley). Dans cette expérience, AcSDKP a été mélangé au Matrigel avant implantation. Sept jours plus tard, les animaux ont été sacrifiés et les échantillons du Matrigel ont été prélevés, fixés dans le formol et on fait l'objet d'une étude histologique. Les observations microscopiques et la quantification des vaisseaux après coloration et immunomarquage ont révélé la présence d'un nombre beaucoup plus important de vaisseaux à l'intérieur du Matrigel ayant contenu AcSDKP par rapport au Matrigel témoin (Tableau 1). Pour la quantification, les sections vasculaires ont été comptées sur 10 champs consécutifs (surface = 32 mm²) à partir de la zone la plus riche.

**Tableau 1**

| Effet de AcSDKP sur l'invasion vasculaire *in vivo* du Matrigel | | | |
|---|---|---|---|
| Traitement | Nombre de vaisseaux (% d'augmentation par rapport au témoin) | | |
| | Exp. 1 | Exp.2 | Exp. 3 |
| AcSDKP 10⁻⁵ M | 60 | 185 | - |
| AcSDKP 10⁻⁶ M | - | 121 | - |
| AcSDKP 10⁻⁷ M | 294 | 118 | 139 |
| AcSDKP 10⁻⁸ M | - | 223 | - |
| AcSDKP 10⁻⁹ M | 108 | 61 | 197 |
| FGFb (50 ng/ml) | - | 297 | - |

### EXEMPLE 5

Compte-tenu de la capacité des facteurs angiogéniques à stimuler *in vivo* la formation de nouveaux vaisseaux dans des régions lésées qui sont caractérisées par un déficit de la vascularisation, nous avons étudié l'efficacité de l'AcSDKP à favoriser la réparation des lésions tissulaires en utilisant un modèle de lambeau cutané. En effet, la nécrose distale de lambeaux cutanés résulte généralement d'une rupture du réseau vasculaire et donc d'une insuffisance de flux artériel, ce que pose d'importants problèmes dans les domaines de la chirurgie esthétique et reconstitutive. Il a été prouvé que l'administration d'un facteur angiogénique, qui contribue à la revascularisation de ces lambeaux, augmente ainsi leur survie.

Nous avons montré que des injections s.c. de AcSDKP dans la région des lambeaux diminuent leurs nécroses. Les lambeaux cutanés ventraux (6 x 6 cm) pédiculés (pédicule inguinal gauche) ont été réalisés chez les rats Sprague-Dawley. AcSDKP a été administré à la dose de 5µg/kg/injection (250 µl/injection) immédiatement après l'intervention chirurgicale, puis encore 5 fois à 12 heures d'intervalle. Les résultats obtenus montrent que la survie des lambeaux chez les animaux traités avec AcSDKP augmente de 10 % par rapport aux contrôles. Sur le plan macroscopique, la diminution de la nécrose est accompagnée par l'augmentation de la densité de la vascularisation sur la surface interne des lambeaux.

## Revendications

1. Utilisation d'un composé de formule I : 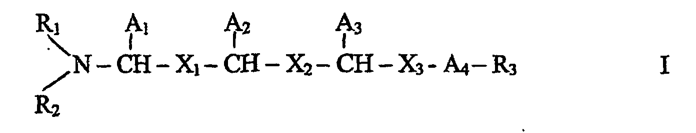 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi H, C₁-C₁₂-alkyle substitué ou non, C₇-C₂₀-arylalkyle substitué ou non, R₄CO ou R₄COO, R₄ étant C₁-C₁₂-alkyle substitué ou non, C₇-C₂₀-arylallcyle substitué ou non, parmi les substitutions il faut citer OH, NH₂ ou COOH,
X₁ et X₂ sont des liaisons peptidiques ou pseudopeptidiques,
X₃ est CO ou CH₂ et
R₃ est OH, NH₂, C₁-C₁₂-alcoxy ou NH-X₄-CH₂-Z, X₄ est un C₁-C₁₂-hydrocarbone normal ou ramifié, Z est H, OH, CO₂H ou CONH₂,
ou bien les tripeptides correspondants comprenant les radicaux A₁, A₂, A₃,
ainsi que les sels pharmaceutiquement acceptables, pour la réalisation d'un médicament destiné au traitement des pathologies pouvant bénéficier d'une angiogenèse.

2. Utilisation selon la revendication 1, **caractérisée en ce que** les pathologies en cause sont des pathologies vasculaires, notamment les ischémies.

3. Utilisation selon la revendication 1, **caractérisée en ce que** les pathologies en cause sont des pathologies impliquant une lésion des tissus, notamment la cicatrisation et la réparation de fractures.

4. Utilisation selon la revendication 1, **caractérisée en ce que** les pathologies en cause impliquent une régénération nerveuse, une chirurgie reconstructrice, une endothélialisation de matériaux ou une transplantation d'organes.

5. Utilisation selon l'une des revendications 1 à 4, **caractérisée en ce que** le composé de formule I est un pseudopeptide dérivé d'un peptide AcSDKP.

6. Utilisation selon l'une des revendications 1 à 4, **caractérisée en ce que** le composé de formule I est un tripeptide dérivé d'un peptide AcSDKP et comprenant les radicaux A₁, A₂, A₃.

7. Utilisation selon l'une des revendications 1 et 6, **caractérisée en ce que** le composé est choisi parmi :
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

8. Utilisation selon l'une des revendications 1 et 6, **caractérisée en ce que** le composé est choisi parmi :
CH₃CO-Ser-Asp-Lys-OH
CH₃CO-Ser-Ψ-(CH₂NH)-Asp-Lys-OH
CH₃CO-Ser-Asp-Ψ-(CH₂NH)-Lys-OH
CH₃CO-Ser-Ψ-(CH₂NH)-Asp-Lys-NH₂
CH₃CO-Ser-Asp-Ψ-(CH₂NH)-Lys-NH₂
H-Ser-Ψ-(CH₂NH)-Asp-Lys-OH
H-Ser-Asp-Ψ-(CH₂NH)-Lys-OH
HOOCCH₂CH₂CO-Ser-Ψ-(CH₂NH)-Asp-Lys-OH
HOOCCH₂CH₂CO-Ser-Asp-Ψ-(CH₂NH)-Lys-OH
H-Ser-Ψ-(CH₂NH)-Asp-Lys-NH₂
H-Ser-Asp-Ψ-(CH₂NH)-Lys-NH₂
HOOCCH₂CH₂CO-Ser-Ψ-(CH₂NH)-Asp-Lys-NH₂
HOOCCH₂CH₂CO-Ser-Asp-Ψ-(CH₂NH)-Lys-NH₂
CH₃CO-Ser-Asp-Lys-NH₂
H-Ser-Asp-Lys-NH₂
CH₃CO-Ser-Asp-Lys-NHCH₃
H-Ser-Asp-Lys-NHCH₃
HOOCCH₂CH₂CO-Ser-Asp-Lys-NHCH₃
HOOCCH₂CH₂CO-Ser-Asp-Lys-NH₂

9. Utilisation d'un composé de formule I : 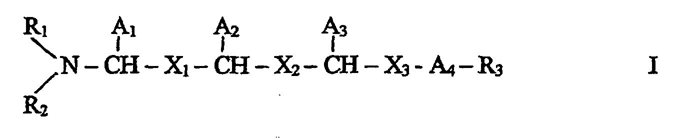 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi H, C₁-C₁₂-alkyle substitué ou non, C₇-C₂₀-arylalkyle substitué ou non, R₄CO ou R₄COO, R₄ étant C₁-C₁₂-alkyle substitué ou non, C₇-C₂₀-arylalkyle substitué ou non, parmi les substitutions il faut citer OH, NH₂ ou COOH,
X₁ et X₂ sont des liaisons peptidiques ou pseudopeptidiques,
X₃ est CO ou CH₂ et
R₃ est OH, NH₂, C₁C₁₂-alcoxy ou NH-X₄-CH₂-Z, X₄ est un C₁-C₁₂-hydrocarbone normal ou ramifié, Z est H, OH, CO₂H ou CONH₂,
ou bien les tripeptides correspondants comprenant les radicaux A₁, A₂, A₃,
ainsi que les sels pharmaceutiquement acceptables,
à titre d'inducteur d'angiogenèse dans les cultures de tissus *ex vivo* ou *in vitro*.

## Patentansprüche

1. Verwendung einer Verbindung der Formel I: 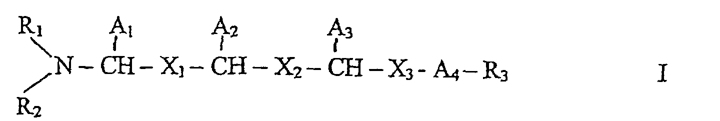 in welcher
A₁ der D- oder L-Ser entsprechende Rest ist,
A₂ der D- oder L-Asp oder -Glu entsprechende Rest ist,
A₃ der D- oder L-Lys, -Arg oder -Orn entsprechende Rest ist,
A₄ der D- oder L-Pro entsprechende Rest ist,
wobei R₁ und R₂ unabhängig voneinander aus H, substituiertem oder nicht-substituiertem C₁-C₁₂-Alkyl, substituiertem oder nicht-substituiertem C₇-C₂₀-Arylalkyl, R₄CO oder R₄COO, wobei R₄ substituiertes oder nicht-substituiertes C₁-C₁₂-Alkyl, substituiertes oder nicht-substituiertes C₇-C₂₀-Arylalkyl ist, ausgewählt werden; unter den Substitutionen müssen OH, NH₂ oder COOH aufgeführt werden;
X₁ und X₂ Peptid- oder Pseudopeptidbindungen sind,
X₃ CO oder CH₂ ist und
R₃ OH, NH₂, C₁-C₁₂-Alkoxy oder NH-X₄-CH₂-Z ist, X₄ ein normaler oder verzweigter C₁-C₁₂-Kohlenwasserstoff ist, Z H, OH, CO₂H oder CONH₂ ist,
oder ebenso der entsprechenden Tripeptide, die die Reste A₁, A₂, A₃ umfassen,
wie auch der pharmazeutisch annehmbaren Salze für die Herstellung eines Arzneimittels, welches für die Behandlung von Pathologien, die aus einer Angiogenese Nutzen ziehen können, bestimmt ist.

2. Verwendung nach Anspruch 1, **dadurch gekennzeichnet, dass** die fraglichen Pathologien vaskuläre Pathologien, insbesondere Ischämien sind.

3. Verwendung nach Anspruch 1, **dadurch gekennzeichnet, dass** die fraglichen Pathologien Pathologien, bei denen eine Läsion von Geweben auftritt, insbesondere Narbenbildung und die Reparatur von Brüchen sind.

4. Verwendung nach Anspruch 1, **dadurch gekennzeichnet, dass** bei den fraglichen Pathologien eine Regeneration von Nerven, eine rekonstruktive Chirurgie, eine Endothelialisierung von Materialien oder eine Transplantation von Organen auftritt.

5. Verwendung nach einem der Ansprüche 1 bis 4, **dadurch gekennzeichnet, dass** die Verbindung der Formel I ein von einem AcSDKP-Peptid abgeleitetes Pseudopeptid ist.

6. Verwendung nach einem der Ansprüche 1 bis 4, **dadurch gekennzeichnet, dass** die Verbindung der Formel I ein Tripeptid ist, das von einem AcSDKP-Peptid abgeleitet ist und die Reste A₁, A₂, A₃ umfasst.

7. Verwendung nach einem der Ansprüche 1 bis 6, **dadurch gekennzeichnet, dass** die Verbindung ausgewählt wird unter:
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

8. Verwendung nach einem der Ansprüche 1 bis 6, **dadurch gekennzeichnet, dass** die Verbindung ausgewählt wird unter:
CH₃CO-Ser-Asp-Lys-OH
CH₃CO-Ser-Ψ-(CH₂NH)-Asp-Lys-OH
CH₃CO-Ser-Asp-Ψ-(CH₂NH)-Lys-OH
CH₃CO-Ser-Ψ-(CH₂NH)-Asp-Lys-NH₂
CH₃CO-Ser-Asp-Ψ-(CH₂NH)-Lys-NH₂
H-Ser-Ψ-(CH₂NH)-Asp-Lys-OH
H-Ser-Asp-Ψ-(CH₂NH)-Lys-OH
HOOCCH₂CH₂CO-Ser-Ψ-(CH₂NH)-Asp-Lys-OH
HOOCCH₂CH₂CO-Ser-Asp-Ψ-(CH₂NH)-Lys-OH
H-Ser-Ψ-(CH₂NH)-Asp-Lys-NH₂
H-Ser-Asp-Ψ-(CH₂NH)-Lys-NH₂
HOOCCH₂CH₂CO-Ser-Ψ-(CH₂NH)-Asp-Lys-NH₂
HOOCCH₂CH₂CO-Ser-Asp-Ψ-(CH₂NH)-Lys-NH₂
CH₃CO-Ser-Asp-Lys-NH₂
H-Ser-Asp-Lys-NH₂
CH₃CO-Ser-Asp-Lys-NHCH₃
H-Ser-Asp-Lys-NHCH₃
HOOCCH₂CH₂CO-Ser-Asp-Lys-NHCH₃
HOOCCH₂CH₂CO-Ser-Asp-Lys-NH₂

9. Verwendung einer Verbindung der Formel I: 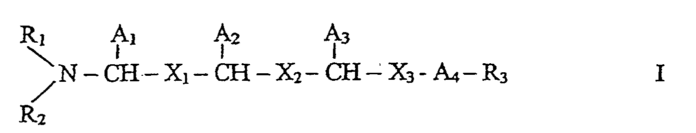 in welcher
A₁ der D- oder L-Ser entsprechende Rest ist,
A₂ der D- oder L-Asp oder -Glu entsprechende Rest ist,
A₃ der D- oder L-Lys, -Arg oder -Orn entsprechende Rest ist,
A₄ der D- oder L-Pro entsprechende Rest ist,
wobei R₁ und R₂ unabhängig voneinander aus H, substituiertem oder nicht-substituiertem C₁-C₁₂-Alkyl, substituiertem oder nicht-substituiertem C₇-C₂₀-Arylalkyl, R₄CO oder R₄COO, wobei R₄ substituiertes oder nicht-substituiertes C₁-C₁₂-Alkyl, substituiertes oder nicht-substituiertes C₇-C₂₀-Arylalkyl ist, ausgewählt werden; unter den Substitutionen müssen OH, NH₂ oder COOH aufgeführt werden;
X₁ und X₂ Peptid- oder Pseudopeptidbindungen sind,
X₃ CO oder CH₂ ist und
R₃ OH, NH₂, C₁-C₁₂-Alkoxy oder NH-X₄-CH₂-Z ist, X₄ ein normaler oder verzweigter C₁-C₁₂-Kohlenwasserstoff ist, Z H, OH, CO₂H oder CONH₂ ist,
oder ebenso der entsprechenden Tripeptide, die die Reste A₁, A₂, A₃ umfassen,
wie auch der pharmazeutisch annehmbaren Salze
als Induktor der Angiogenese in ex vivo- oder *in vitro*-Gewebekulturen.

## Claims

1. Use of a compound of formula I: 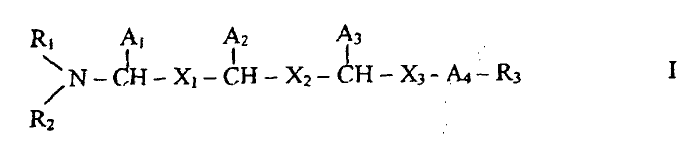 in which,
A₁ is the radical corresponding to D- or L- Ser,
A₂ is the radical corresponding to D- or L- Asp or Glu,
A₃ is the radical corresponding to D- or L- Lys, Arg or Orn,
A₄ is the radical corresponding to D- or L- Pro,
R₁ and R₂ are chosen, independently, from H, C₁-C₁₂-alkyl which may or may not be substituted, C₇-C₂₀-arylalkyl which may or may not be substituted, R₄CO or R₄COO, R₄ being C₁-C₁₂-alkyl which may or may not be substituted, or 'C₇-C₂₀-arylalkyl which may or may not be substituted; among the substitutions, mention should be made of OH, NH₂ or COOH,
X₁ and X₂ are peptide or pseudopeptide bonds,
X₃ is CO or CH₂ and
R₃ is OH, NH₂, C₁-C₁₂-alkoxy or NH-X₄-CH₂-Z, X₄ is a normal or branched C₁-C₁₂ hydrocarbon, and Z is H, OH, CO₂H or CONH₂,
or the corresponding tripeptides comprising the radicals A₁, A₂, A₃,
and also the pharmaceutically acceptable salts, for preparing a medicinal product intended for the treatment of pathologies which may benefit from angiogenesis.

2. Use according to Claim 1, **characterized in that** the pathologies in question are vascular pathologies, in particular ischaemias.

3. Use according to Claim 1, **characterized in that** the pathologies in question are pathologies which involve a tissue lesion, in particular fracture cicatrization and repair.

4. Use according to Claim 1, **characterized in that** the pathologies in question involve nerve regeneration, reconstructive surgery, endothelialization of material or organ transplantation.

5. Use according to one of Claims 1 to 4, **characterized in that** the compound of formula I is a pseudopeptide derived from a peptide AcSDKP.

6. Use according to one of Claims 1 to 4, **characterized in that** the compound of formula I is a tripeptide derived from a peptide AcSDKP and comprising the radicals A₁, A₂, A₃.

7. Use according to either of Claims 1 and 6, **characterized in that** the compound is chosen from:
CH₃CO-Ser-Asp-Lys-Pro-OH
CH₃CO-Ser-ψ-(CH₂NH)-Asp-Lys-Pro-OH
CH₃CO-Ser-Asp-ψ-(CH₂NH)-Lys-Pro-OH
CH₃CO-Ser-Asp-Lys-ψ-(CH₂N)-Pro-OH
CH₃CO-Ser-ψ-(CH₂NH)-Asp-Lys-Pro-NH₂ CH₃CO-Ser-Asp-ψ-(CH₂NH)-Lys-Pro-NH₂
CH₃CO-Ser-Asp-Lys-ψ-(CH₂N)-Pro-NH₂
H-Ser-ψ-(CH₂NH)-Asp-Lys-Pro-OH
H-Ser-Asp-ψ-(CH₂NH)-Lys-Pro-OH
H-Ser-Asp-Lys-ψ-(CH₂N)-Pro-OH
HOOCCH₂CH₂CO-Ser-ψ-(CH₂NH)-Asp-Lys-Pro-OH
HOOCCH₂CH₂CO-Ser-Asp-ψ-(CH₂NH)-Lys-Pro-OH
HOOCCH₂CH₂CO-Ser-Asp-Lys-ψ-(CH₂N)-Pro-OH
H-Ser-ψ-(CH₂NH)-Asp-Lys-Pro-NH₂
H-Ser-Asp-ψ-(CH₂NH)-Lys-Pro-NH₂
H-Ser-Asp-Lys-ψ-(CH₂N)-Pro-NH₂
HOOCCH₂CH₂CO-Ser-ψ-(CH₂NH)-Asp-Lys-Pro-NH₂
HOOCCH₂CH₂CO-Ser-Asp-ψ-(CH₂NH)-Lys-Pro-NH₂
HOOCCH₂CH₂CO-Ser-Asp-Lys-ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

8. Use according to either of Claims 1 and 6, **characterized in that** the compound is chosen from:
CH₃CO-Ser-Asp-Lys-OH
CH₃CO-Ser-ψ-(CH₂NH)-Asp-Lys-OH
CH₃CO-Ser-Asp-ψ-(CH₂NH)-Lys-OH
CH₃CO-Ser-ψ-(CH₂NH)-Asp-Lys-NH₂
CH₃CO-Ser-Asp-ψ-(CH₂NH)-Lys-NH₂
H-Ser-ψ-(CH₂NH)-Asp-Lys-OH
H-Ser-Asp-ψ-(CH₂NH)-Lys-OH
HOOCCH₂CH₂CO-Ser-ψ-(CH₂NH)-Asp-Lys-OH
HOOCCH₂CH₂CO-Ser-Asp-ψ-(CH₂NH)-Lys-OH
H-Ser-ψ-(CH₂NH)-Asp-Lys-NH₂
H-Ser-Asp-ψ-(CH₂NH)-Lys-NH₂
HOOCCH₂CH₂CO-Ser-ψ-(CH₂NH)-Asp-Lys-NH₂
HOOCCH₂CH₂CO-Ser-Asp-ψ-(CH₂NH)-Lys-NH₂
CH₃CO-Ser-Asp-Lys-NH₂
H-Ser-Asp-Lys-NH₂
CH₃CO-Ser-Asp-Lys-NHCH₃
H-Ser-Asp-Lys-NHCH₃
HOOCCH₂CH₂CO-Ser-Asp-Lys-NHCH₃
HOOCCH₂CH₂CO-Ser-Asp-Lys-NH₂

9. Use of a compound of formula I: 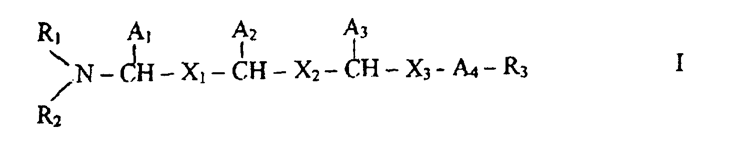 in which,
A₁ is the radical corresponding to D- or L- Ser,
A₂ is the radical corresponding to D- or L- Asp or Glu,
A₃ is the radical corresponding to D- or L- Lys, Arg or Orn,
A₄ is the radical corresponding to D- or L- Pro,
R₁ and R₂ are chosen, independently, from H, C₁-C₁₂-alkyl which may or may not be substituted, C₇-C₂₀-arylalkyl which may or may not be substituted, R₄CO or R₄COO, R₄ being C₁-C₁₂-alkyl which may or may not be substituted, or C₇-C₂₀-arylalkyl which may or may not be substituted; among the substitutions, mention should be made of OH, NH₂ or COOH,
X₁ and X₂ are peptide or pseudopeptide bonds,
X₃ is CO or CH₂ and
R₃ is OH, NH₂, C₁-C₁₂-alkoxy or NH-X₄-CH₂-Z, X₄ is a normal or branched C₁-C₁₂ hydrocarbon, and Z is H, OH, CO₂H or CONH₂,
or the corresponding tripeptides comprising the radicals A₁, A₂, A₃,
and also the pharmaceutically acceptable salts, as an angiogenesis inducer in ex vivo or *in vitro* tissue cultures.
